# EUROPEAN PATENT APPLICATION

(11) **EP 1 136 048 A1**
(43) Date of publication of application: **26.09.2001**
(21) Application number: 00105380.0
(22) Date of filing: 21.03.2000
(51) Int. Cl.: A61F 13/15

(54) **Multiple component incontinence care system : diaper and human waste collector**

(71) Applicant: THE PROCTER & GAMBLE COMPANY, Cincinnati, Ohio 45202 (US)
(72) Inventor: D'Acchioli, Vincenzo, 65779 Kelkheim am Taunus (DE); Palumbo, Gianfranco, 61348 Bad Homburg (DE)
(74) Representative: Kohol, Sonia

(57) **Abstract**

The present invention relates to an incontinence care system and the use thereof which is suitable for the collection of urine and/or faeces and/or menses. Claimed and described is an incontinence care system comprising at least two components selected from the group of human waste collection bag, absorbent pad, partial diaper (350), orificed diaper (450) and orificed incontinence garment for adult or infant incontinence.

## Description

### Field of the invention

The present invention relates to an incontinence care system suitable for the collection of urine and/or faeces and/or menses. The system comprises at least two individual components, for example an absorbent structure and a human waste collection bag.

### Background of the invention

Human waste collection bags, principally comprising faecal collection bags and urine collection bags, are known articles of manufacture that are designed to be worn principally by incontinence sufferers and in particular by bedridden patients. Such human waste collection bags are attached to the perianal or uro-genital region of the wearer and are intended to entrap and immediately contain faeces, urine and other bodily discharges.

Representative prior art in the field of faecal collection bags includes the following documents: US 3,734,096 and US 3,522,807 disclose faecal collection bags provided with adhesive tabs to support the attachment of the collection bag to the human body. GB 2 152 387, GB 2 215 605, SE 8 104 934 and EP 245 064 all disclose faecal collection bags which are made from plastic materials and rely on an principally flat adhesive flange for attachment to and seal with the human body. JP 8-117261 discloses a faecal collection bag, which is attached to the body by sheets of material cut to surround the lower parts of the body similar to a piece of underwear. US 4,784,656 discloses a two piece faecal collection bag. The piece providing the collection capacity can be emptied or replaced while the other piece remains engaged in body contact.

Representative disclosures of urine collection bags are, for instance, found in the following documents: US 4,804,377 discloses a collection bag for urine specimens from children. US 3,292,626 discloses a urine collection bag for female infants.

Some prior art documents disclose devices for multiple incontinence: For example, US 3,577,989 discloses a device for female users comprising a plastic bag for the collection of both, urine and faeces. The bag is provided with a flange portion to cover the urinary duct and the anus, the flange comprising elastic straps for attachment to the body. US 3,532,093 discloses a collector for urine and faeces comprising two compartments for the respective collection of urine and faeces. Other devices for multiple incontinence are conventional diapers as provided for babies and for incontinent adults.

Some documents, both in the field of urine collection bags and faecal collection bags, disclose devices which are provided with humps so as to achieve a better seal with the human body. GB 1,092,274 discloses a wedge-like projection on an urine collection bag. Similarly GB 1,092,274 discloses a pediatric urine collection bag for female use, where the base of the bag opening is provided with a wedge like projection adapted to engage the lower perineal area of the infant. European patent application 97110604.2 and international application PCT/US98/13360 disclose faecal collection bags which are provided with projections/humps to fit the perineum and/or the coccyx, thereby ensuring a much improved fit and sealing of the respective devices.

A considerable group of users of incontinence devices is both, urine and faecal incontinent. Those user's namely include babies, but also unhealthy and elderly people. The degree of incontinence is highly individual and may be different with regard to the different kinds of incontinence, i.e. urine or faecal. Also some users may have a need for devices which are suitable for faecal and/or urine incontinence and further suitable for collecting menses. Those needs are not only highly individual, they may further be time dependent, e.g. be different between day time and night time.

Prior art devices provided to address multiple incontinence as disclosed above are one piece devices. Such devices, however, need to be changed as a whole if either their capacity for urine or their capacity for faeces is used up irrespective of capacity left for the respective other exudate. Hence, there is a need for a device whose capacities can be used more effectively and thereby more economically and ecologically. Moreover, there is a need for a device which can be adapted to suit the above individual incontinence needs.

Another problem well known in the art is that of the proper placement of a human waste collection bag, again in particular for those devices designed for adhesive attachment to the wearer's skin. Total or substantial misplacement of the device will lead to a severe misfunctioning, may it be leaking or may it even be unintentional detachment. Devices provided with humps provide substantially improved fit and seal. However, these devices typically cannot be worn in combination with other incontinence devices, since the hump takes up space in the perineal area and makes the wearing of another device extending into the perineal area highly inconvenient.

In view of the prior art there remains a need for an incontinence device which:
- provides improved urine and faecal incontinence care
- optionally provides for feminine hygienic care
- independently provides absorbent capacity for the above tasks adaptable to individual needs at a given time
- allows to separately remove and attach components for urine incontinence, faecal incontinence or feminine hygienic care
- is environmentally friendly
- is comfortable to wear, for bedridden, but also for active wearer's
- engages in reliable seal with the human body
- can easily be positioned and applied

These and other objectives are addressed by the present invention as apparent from the following description.

### Summary of the invention

The present invention relates to an incontinence care system and the use thereof which is suitable for the collection of urine and/or faeces and/or menses. Claimed and described is an incontinence care system comprising at least two components selected from the group of human waste collection bag, absorbent pad, partial diaper (350), orificed diaper (450) and orificed incontinence garment. A human waste collection bag is either a faecal collection bag (110) or a urine collection bag (210). Particularly claimed is the use of at least two and preferably two components selected from the above group for adult or infant incontinence.

### Brief description of the drawings

It is believed that the invention will be better understood from the foregoing description in conjunction with the accompanying drawings in which:

Figure 1 is a perspective view of a preferred embodiment of a faecal collection bag. L denotes the longitudinal direction and T denotes the transversal direction.

Figure 2 is a top view of a preferred embodiment of a urine collection bag. L denotes the longitudinal direction and T denotes the transversal direction.

Figure 3 is a partially cut-away perspective view of a partial diaper to be worn in combination with a faecal collection bag according to the present invention.

Figure 4 is a partially cut-away perspective view of an orificed diaper to be worn in combination with a faecal collection bag according to the present invention.

### Detailed description of the invention

The present invention relates to a incontinence care system suitable for the collection of urine and/or faeces and/or menses. The term "system", as used herein, comprises a kit comprising at least two, preferably two components and the use of at least two, preferably two components. The term "kit", as used herein, refers to a multitude of components, provided together in any form, preferably by providing them in a common package or alternatively by providing at least one component with usage instructions referring to at least one other component.

A multiple component incontinence care system according to the present invention comprises at least two components selected from the group of human waste collection bag, absorbent pad, partial diaper (350), orificed diaper (450) and orificed incontinence garment, as described hereinafter. The term "human waste collection bag", as used herein, denotes either a faecal collection bag (110) or a urine collection bag (210). Preferred components according to the present invention comprise the following:
- A diaper as described hereinafter having a front and a rear portion provided with reduced or free of absorbent means in said front or rear portion which corresponds to the anal or uro-genital area compared to other areas of the diaper, for combination with a urine collection bag (210) or a faecal collection bag (110), respectively, herein referred to as "partial diaper (350)".
- Further the diaper may be provided with an orifice (84) extending from the topsheet (56) to the backsheet (60) for attachment of a human waste collection bag to the skin of the wearer through the orifice, herein referred to as "orificed diaper (450)". An orificed diaper allows to insert at least the flange (12) of a human waste collection bag or optionally further portions of the human waste collection bag through the orifice. The orifice is located in the front or rear portion of the diaper depending on whether the orificed diaper (450) is to be combined with a faecal collection bag(110) or a urine collection bag (210).
- An incontinence garment provided with an orifice through which a human waste collection bag can be adhesively attached to the body and preferably also with a flange surrounding the orifice to connect to a human waste collection bag, hereinafter referred to as orificed incontinence garment.

According to the present invention it is particularly preferred that the components are used in combination as follows:
- An adapted diaper is worn in combination with a urine collection bag (210). The term "adapted diaper", as used herein, comprises partial diapers (350) and orificed diapers (450). For combination with an urine collection bag (210) the adapted diaper can be provided either as an orificed diaper (450) with an orifice in the front portion adjacent to the uro-genital area or as a partial diaper (350) with a reduced amount of absorbent material in the front portion adjacent to the uro-genital area of a wearer.
- An adapted diaper is worn in combination with a faecal collection bag (110). The adapted diaper can be provided either as an orificed diaper (450) with an orifice in the rear portion adjacent to the perianal area or as a partial diaper (350) with a reduced amount of absorbent material in the rear portion adjacent to the perianal area of a wearer.
- An absorbent pad is used in combination with a faecal collection bag. Preferably the absorbent pad is adapted to have a reduced amount of absorbent material adjacent to perineal area of a wearer.

The adaptations made to the components in accordance with the present invention and the components themselves will be described in more detail hereinafter.

### The human waste collection bag

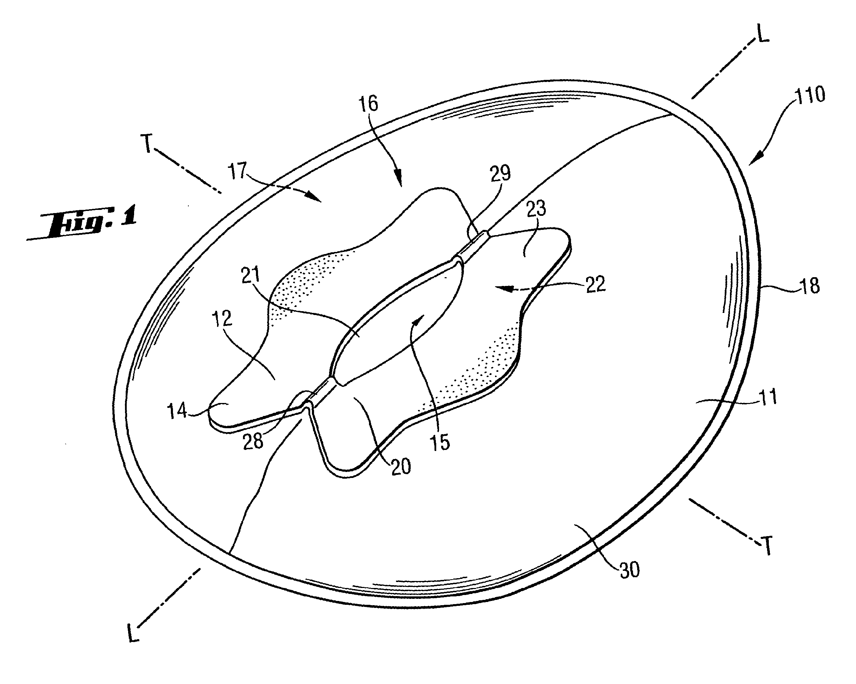

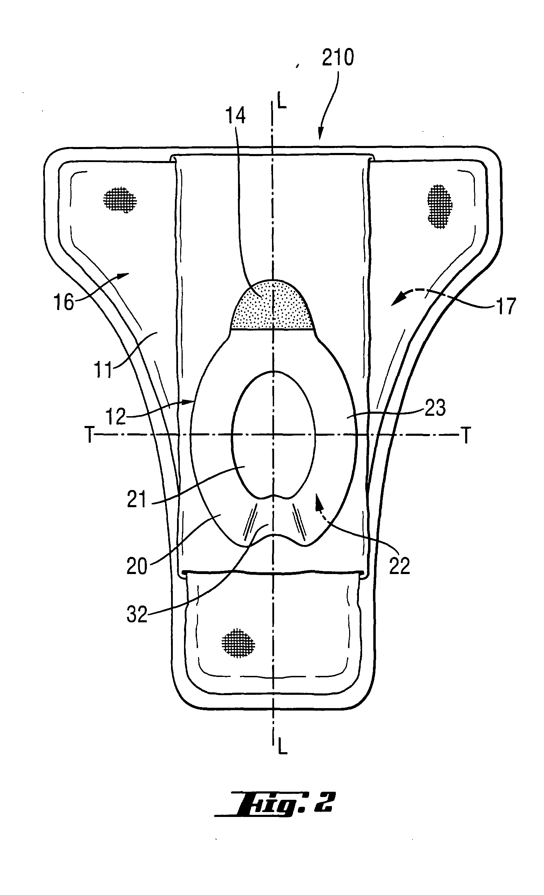

Human waste collection bags comprise faecal collection bags (110) and urine collection bags (210). Both types of collection bags can comprise similar or alike components and materials and are therefore described together. A typical faecal collection bag (110) is shown in Figure 1 and a typical urine collection bag (210) is shown in Figure 2. Faecal collection bags (110) are designed for attachment to the anal area and mainly used for collecting faeces, whereas urine collection bags (210) are attached to the urinary duct and mainly used for collecting urine. All of the above human waste collection bags are preferably designed for single use and disposal thereafter.

Typically human waste collection bags comprise a bag (11) having an aperture (21) and a flange (12) surrounding the aperture for preferably adhesive attachment to the perianal area of a wearer as visible from Figure 1. Any human waste collection bag known in the art can be provided according to the present invention.

The bag (11), as used herein, is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

According to the present invention the bag material can comprise one or multiple layers, preferably two or three layers. The layer on the inside of the bag (11), which will typically at least partially come in contact with faecal material or urine is called the inner layer. The outermost layer of the bag, which will typically at least partially come in contact with the skin to the wearer and the garments of the wearer, is called the outer layer.

The layers of the bag material may comprise any material, preferably so that the bag is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose-wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag material may comprise a non-woven layer. Such material layers present an uneven surface to the skin of the wearer and thus reduce significantly the problem of occlusion and greatly improve skin healthiness.

In one preferred embodiment of the present invention the bag material comprises two layers. Preferably the outer layer comprises a non-woven layer and the inner layer comprises a film.

In yet another preferred embodiment of the present invention, the bag material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag material may be hydrophobic or hydrophilic. If the bag material does not comprise a film layer, preferably at least one non-woven layer is hydrophobic. As a consequence, fluid penetration is resisted through the wearer facing portion (16) and the garment facing portion (17) of the human waste collection bag (10). If the bag material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, cationic, non-ionic and amphoteric surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag is within the scope of the present invention. Particularly, preferred shapes are flat circular type bags, cone shaped bags, truncated cone shaped bags and pyramidal or truncated pyramidal shaped bags and flat T shaped bags. For faecal collection bags (110) the truncated cone shape is most preferred, whereas for urine collection bags (210) the flat T-shape is most preferred.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

The bag (11) is provided with an aperture (21) whereby faecal matter or urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (14).

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m³, more preferably 50 g/m³. Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

According to the present invention the human waste collection bag (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (14) on either side of the flange (12) which are non-adhesive and can thereby serve to facilitate placement and removal of the device whilst avoiding contact with the adhesive. These lobes (14) are however preferably also covered by the release means. Before application of the human waste collection bag (10) to the skin of the wearer, the release means if present is removed.

According to the present invention any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste collection bags (10) to be used for babies the amount of adhesive (20) may be less than for human waste collection bags (10) designed for active adult incontinence sufferers.

The flange (12) comprises a garment facing portion (22) and a wearer facing portion (23). These are two large surfaces, which may be flat or may extend into three dimensions, however, the flange (12) may also comprise projections, a front projection and/or a rear projection, also referred to as front and rear hump, respectively, in case of a faecal collection bag (110) designed to fit the perineal and/or coccygeal area of the wearer and in case of a urine collection bag (10) designed to fit the genital and/or perineal area. Figure 1 shows a faecal collection bag (110) provided with a front projection (28) and a rear projection (29). Figure 2 shows a urine collection bag (210) provided with a projection (32) (also referred to as bulge or hump) to fit the perineum.

According to the present invention a multiple component incontinence care system may comprise at least one human waste collection bag, and in one preferred embodiment is provided from one faecal collection bag and one urine collection bag (210). According to the present invention only one of these collection bags should be provided with a projection to fit the perineum. Preferably the faecal collection bag is provided with such perineal protection since sealing of a faecal collection bag is more critical, because faeces potentially build up more pressure towards the perineum, especially when a wearer is sitting.

### The absorbent pad

The present invention contemplates a variety of absorbent pads, included those typically referred to as sanitary napkins, panty liners and incontinence pads.

The absorbent pad comprises a topsheet, a backsheet, an absorbent core and has longitudinal side margins and lateral side margins.

### The topsheet

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in US 3,929,135; US 4,324,246; US 4,342,314; US 4,463,045; and US 5,006,394. Particularly preferred micro apertured formed film topsheets are disclosed in US 4,609,518 and US 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets not having a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in US 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet typically extends across the whole of the absorbent structure and outside the area coextensive with the absorbent structure. The topsheet can extend and form part or all of the preferred side flaps.

When referring to the topsheet a multi layer structure or a mono layer structure is contemplated. The hybrid topsheet mentioned above is such a multi layer design but other multi layer topsheets such as primary and secondary topsheet designs are also considered.

### Absorbent core

According to the present invention the absorbent cores suitable for use in herein may be selected from any of the absorbent cores or core system known in the art. As used herein, the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a. Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b. Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c. Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included.

However, this "dusting" layer provides some additional fluid-handling capabilities such as rapid wicking of fluid along the length of the pad.

### d. Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents.

### Backsheet

The backsheet primarily prevents the extrudes absorbed and contained in the absorbent structure from wetting articles that contact the absorbent product such as underpants, pants, pyjamas and, undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance.

Further, the backsheet can permit vapours to escape from the absorbent structure, i.e. be breathable, while still preventing extrudates from passing through the backsheet. Also breathable backsheets comprising several layers, e.g. film plus non-woven structures, can be used. Such backsheets thus comprise at least one gas permeable layer. Suitable gas permeable layers include 2-dimensional, planar micro and macro-porous films, macroscopically expanded films, formed apertured films and monolithic films. The apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein, the term 2 dimensional planar layer refers to layers having a depth of less than 1 mm, preferably less than 0.5 mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane™ available from B.F. Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet component comprises a microporous film and an apertured formed film or a microporous and a hydrophobic woven or nonwoven material.

The absorbent pad preferably also has fasteners for securing the absorbent pad in place in a wearer's undergarment. The fasteners used with the absorbent pad are not limited to adhesive fasteners. Any suitable type of fastener known in the art can be used for this purpose. For example, the absorbent pad could be secured in place in a wearer's undergarment by mechanical fasteners, such as VELCRO™ or by a combination of adhesive and mechanical fasteners. For simplicity, however, the fasteners will be described in terms of adhesive fasteners and these fasteners are preferably pressure sensitive adhesive fasteners. Suitable pressure sensitive adhesive fasteners are described in greater detail in US 4,917,697.

The panty fastening adhesive can be provided in any suitable configuration. Typically the panty fastening adhesive is positioned on the backsheet of the sanitary napkin. In a preferred embodiment, the panty fastening adhesive is provided in the form of a longitudinally oriented strip of adhesive that is centred about the principal longitudinal centreline. The panty fastening adhesive provides an adhesive attachment means for securing the main body portion of the absorbent pad in the crotch portion of a panty.

Optionally the absorbent pad may comprise side flaps for attachment to e.g. a panty.

According to the present invention the absorbent pad is preferably adapted to be worn in combination with another incontinence component of the present invention as described herein, typically a faecal collection bag. Preferably the absorbent pad is provided with no absorbent means or a reduced amount of absorbent means in the area adjacent to the perineal area. Thereby the product can be made thin and if more comfortable to wear is overlapping another incontinent component, such as a faecal collection bag, in the perineal area.

Alternatively the absorbent pad may be provided with an orifice extending from the topsheet to the backsheet of the absorbent pad. Such orifice allows for the insertion of a urine collection bag (210). Thereby urine and menses can be collected in a very reliable way.

### The adapted diaper

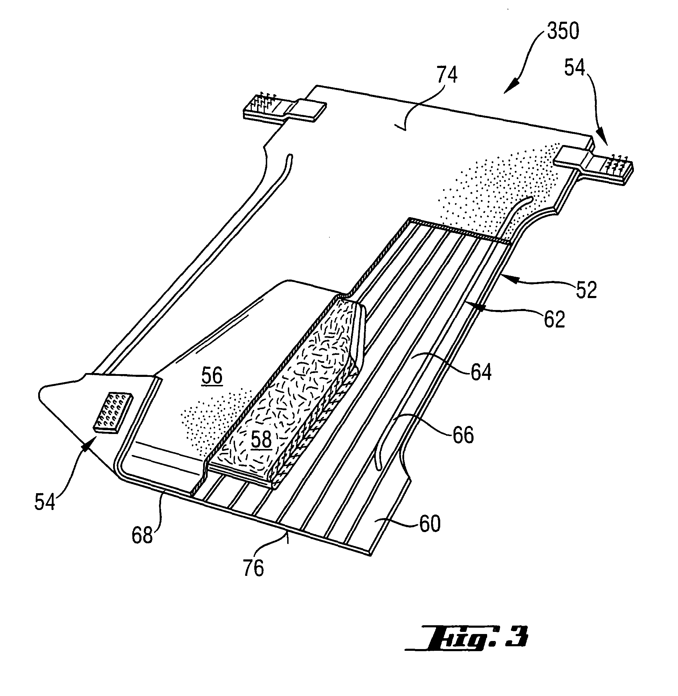

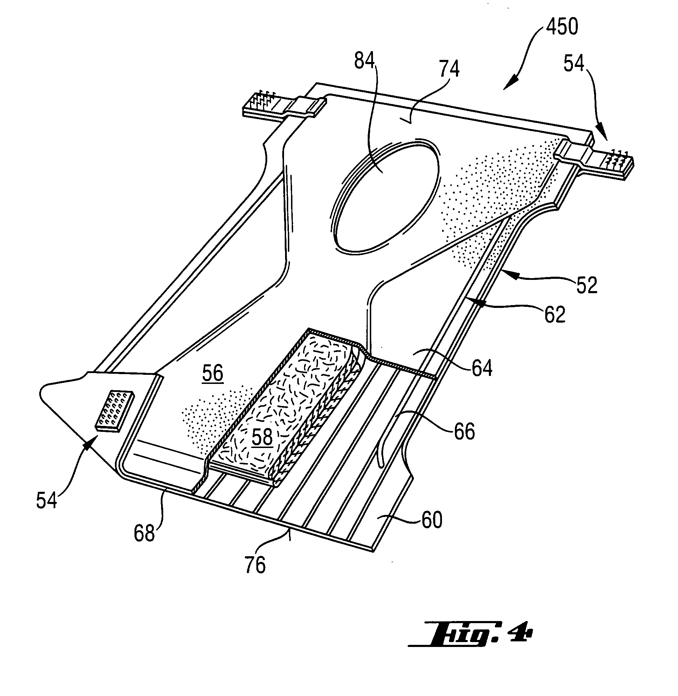

The human waste collection bag of the present invention has been found to be particularly useful and beneficial when used in conjunction with an adapted diaper, either a partial diaper (350) or an orificed diaper (450), refer to Figures 3 and 4, which preferably is disposable. In the following paragraphs reference is made to a faecal collection bag (110), however, a urine collection bag (210) is equally suitable for the combination with an adapted diaper. In particular, the adapted diaper is positioned over the faecal collection bag (110) and fastened in a conventional manner around the body of the wearer. It has been found that, in addition, to providing excellent separation between urine and faecal material, the combination of a faecal collection bag (110) and an adapted diaper actually reduces skin irritation, which may at times occur, especially since the group of typical wearers includes the very old, the very young and the unhealthy wearers.

As used herein, the term "disposable", as used for disposable diapers refers to articles which absorb and contain body exudates; and more specifically, refers to articles which are placed against or in proximity to the body of the wearer to absorb and contain the various exudates discharged from the body and which are intended to be discarded after a single use (i. e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

Figure 3 is a partially cut-away perspective view of a partial diaper (350) embodying the present invention prior to it being placed on the wearer over the faecal collection bag (110). As is visible from Figure 3, a preferred adapted diaper comprises a body portion (52) and a refastenable mechanical fastening device (54). A preferred body portion (52) comprises a liquid pervious topsheet (56), and absorbent core (58), a liquid impervious backsheet (60), and elastically contractible leg cuffs (62); each leg cuff (62) preferably comprising a side flap (64) and one or more elastic members (66). For simplicity purposes, only one elastic member (66) is shown in the side flap (64). While the topsheet (56), the absorbent core (58), the backsheet (60), the side flaps (64), and the elastic members (66) may be assembled in a variety of well-known configurations. A preferred disposable diaper configuration is shown and generally described in US 3,860,003, an even more preferred disposable diaper configuration is shown and generally described in WO 93/16669 (the preferred disposable diapers, however, do not comprise the adaptations of the present invention). In this preferred diaper configuration, the backsheet (60) is joined to the topsheet (56); the absorbent core (58) is positioned between the topsheet (56) and the backsheet (60); the side flaps (64) extend outwardly from and along each side edge of the absorbent core (58); and the elastic member (66) is operatively associated with each side flap (64).

Figure 3 shows the body portion (52) in which the topsheet (56) and the backsheet (60) are coextensive and have length and width dimensions generally larger than those of the absorbent core (58). The topsheet (56) is superposed on the backsheet (60) thereby forming the periphery (68) of the body portion (52).

The body portion (52) has an inside surface (74) and an outside surface (76). When a backsheet (60) is used, it typically forms the outside surface (76) of the body portion (52). The inside surface (74) is that surface of the adapted diaper opposite the outside surface (76) and in the embodiment shown is typically formed by the topsheet (56). In general, the inside surface (74) of the adapted diaper is that surface coextensive with the outside surface (76) and which is for the greater part in contact with the wearer when the adapted diaper is worn.

The backsheet (60) is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (60) prevents the exudates absorbed and contained in the absorbent core (58) from soiling articles which are in contact with the adapted diaper such as undergarments and bedding. The backsheet (60) may thus comprise polymeric films such as thermoplastic films of polyethylene or polypropylene, or composite materials such as film-coated non-woven material. Exemplary films are manufactured by Tredegar Industries, Inc. of Terre Haute, Ind., USA or BP-Chemical PlasTec, Rotbuchenstrasse 1, D-8000 München, Germany.

The backsheet (60) is preferably textured to provide a more clothlike appearance. Further, the backsheet (60) may also permit vapours to escape from the absorbent core (58) while still preventing exudates from passing through the backsheet (60) by, for example, being supplied with microapertures. The size of the backsheet (60) is dictated by the size of the absorbent core (58) and the exact diaper design selected.

The topsheet (56) of the diaper is compliant, soft feeling and non-irritating to the skin of the wearer. Further, the topsheet (56) is liquid pervious permitting liquids (for example, urine) to readily penetrate through its thickness. A suitable topsheet (56) may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core (58).

There are a number of manufacturing techniques which may be used to manufacture the topsheet (56). For example, the topsheet (56) may be a non-woven web of fibres. An exemplary topsheet (56) is carded and thermally bonded by means well-known to those skilled in the fabric art. A suitable topsheet (56) is manufactured by, for example, Veratec Inc., a division of International Paper Company, of Walpole, Mass., USA. A topsheet (56) particularly preferred for incontinence garments comprises a formed thermoplastic film.

The absorbent core (58) of the body portion (52) may be any absorbent means which is generally compressible, conformable, non-irritating to the skin of the wearer, and capable of absorbing and retaining liquids such as urine and other certain bodily discharges. The absorbent core (58) may be manufactured in a variety of sizes and shapes (for example, rectangular, hour-glass or half hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core (58) may also be varied (for example, the absorbent core (58) may have varying caliper zones, hydrophilic gradients, superabsorbent gradients, or lower average density and lower average basis weight acquisition zones; or may comprise one or more layers or structures). Further, the size and absorbent capacity of the absorbent core (58) may be varied to accommodate wearers ranging from infants to adults.

A diaper according to the present invention may be provided as a partial diaper (350), hence with less or free of absorbent material in either the front area for combination with a urine collection bag (210) or the rear area for combination with a faecal collection bag (110).This may be the uro-genital or the perianal area or both areas. Since faecal matter, urine and other bodily exudates are reliably collected by the respective collection bags, absorbent capacity is not needed adjacent to those areas. For example in the embodiment of Figure 3 the absorbent core (58) does not extend into the perianal area. Using no or less absorbent core material in those areas allows to design a thinner product, which is more comfortable to wear. A product with no absorbent core material in both areas gives the benefits of additional attachment of the worn human waste collection bag and additional leakage protection.

As shown in Figure 4 a diaper according to the present invention may also be provided as an orificed diaper (450). The component of the orificed diaper (450) shown in Figure 4 are substantially the same as shown in Figure 3 and described with regard to the partial diaper (350), except for the form of the absorbent core (58) and the adaptations made to provide an orifice (84). The orifice (84) extends from the topsheet (56) to the backsheet (60) for attachment of a human waste collection bag to the skin of the wearer. The orifice (84) may be located in the front portion adjacent to the uro-genital area for attachment of a urine collection bag (210) or may be located in the rear portion adjacent to the perianal area for attachment of a faecal collection bag (110). Such orifice (84) may be of any size or shape. Preferably it has a round or oval shape. For a diaper to be worn by a baby the shortest diameter for such orifice (84) is preferably at least 2 cm, more preferably at least 4 cm, most preferably at least 6 cm. The longest diameter of the orifice (84) is preferably no more than 20 cm, more preferably no more than 15 cm, most preferably no more than 10 cm. Preferably the topsheet (56) and the backsheet (60) are joined by means known in the art to provide the periphery of the orifice (84). Such means include thermobonding, hot and cold crimping and adhesives. While in one embodiment of the present invention the orifice (84) is used to access the body for adhesive attachment of a human waste collection bag, in other embodiments the orifice (84) is equipped with a fastening means for the human waste collection bag. Suitable fastening means include adhesives, a hook-loop system (Velco® ) and any mechanical joining system. Such a fastening system reduces stress on a user's skin when changing the human waste collection bag. Optionally the wearer facing side of the diaper may be positioned with a body compatible adhesive adjacent to the orifice (84).

When a partial diaper (350) is used, the human waste collection bag is preferably first applied to the body and the partial diaper (350) is applied thereafter, typically at least partially covering the human waste collection bag. When an orificed diaper (450) is used, the human waste collection bag and the orificed diaper (450) may be applied in any sequence. The orifice (84) comprised by the partial diaper (50) allows attaching a human waste collection bag to the body there-through. Alternatively the orifice (84) comprised by the partial diaper (50) allows transferring parts of the human waste collection bag, preferably the bag (11), as to wear those parts outside of the orificed diaper (450) thereby potentially increasing wearing comfort. If the orifice (84) is provided with fastening means for a human waste collection bag, the human waste collection bag may be attached to the orificed diaper (450) before the diaper is placed or while the diaper is placed.

### Orificed incontinence garment

An incontinence garment to be used in accordance with the present invention is to be understood as a piece of underwear, similar to a pair of underpants or a panty, adapted to be used with at least one incontinence component, preferably at least one human waste collection bag. The incontinence garment may be designed for disposal after single use or alternatively may be designed to be reuseable. According to the present invention the incontinence garment is provided with at least one orifice, herein referred to as "orificed incontinence garment", the orifice allowing for attachment of a human waste collection bag to the body or to the garment as described above for an orificed diaper (450). Preferably the orifice is surround by a flange which also for attachment of a human waste collection bag. Such attachment may be achieved by a mechanical connector, a hook/loop ensemble such as Velcro™ or adhesive attachment. One preferred means to achieve adhesive attachment is to provide adhesive on the flange (12) of the human waste management device and a compatible adhesive or no adhesive on the flange of the incontinence garment.

## Claims

1. Use of at least two components selected from the group of human waste collection bag, absorbent pad, partial diaper (350), orificed diaper (450) and orificed incontinence garment for adult or infant incontinence.

2. Use of at least two components according to Claim 1, wherein one component is a human waste collection bag and said human waste collection bag is a faecal collection bag (110).

3. Use of at least two components according to Claim 1, wherein one component is a human waste collection bag and said human waste collection bag is a urine collection bag (210).

4. Use of at least two components according to Claim 2, wherein one component is selected from the group of said partial diaper (350) and said orificed diaper (450).

5. Use of at least two components according to Claim 3, wherein one component is selected from the group of said partial diaper (350) and said orificed diaper (450).

6. A kit comprising at least two of the following components: human waste collection bag, absorbent pad, partial diaper (350), orificed diaper (450) and orificed incontinence garment for adult or infant incontinence.
